# EUROPEAN PATENT APPLICATION

(11) **EP 4 535 362 A1**
(43) Date of publication of application: **09.04.2025**
(21) Application number: 24199628.9
(22) Date of filing: 11.09.2024
(51) Int. Cl.: G16B 20/20, G16B 25/10, G16B 40/10

(54) **METABOLITE ANALYSIS SUPPORT SYSTEM**

(30) Priority: 02.10.2023 JP 2023171601
(71) Applicant: SHIMADZU CORPORATION, Kyoto-shi, Kyoto 604-8511 (JP)
(72) Inventor: NAKAGAWA, Yuki, Nakagyo-ku, Kyoto-shi, 604-8511 (JP)
(74) Representative: Müller-Boré & Partner Patentanwälte PartG mbB

(57) **Abstract**

A metabolite analysis support system includes: an enzyme information storage unit for storing enzyme information representing a relation between an enzyme and metabolites for one or more enzymes involved in metabolic pathways in a body of an organism; an analysis condition storage unit for storing an analysis condition for analyzing the metabolites with a mass spectrometer; a gene locus information input unit for inputting information regarding a gene locus affecting an expression of an enzyme of interest; a metabolite identification unit for identifying the metabolites as analysis target metabolites, based on information regarding the gene locus and information stored in the enzyme information storage unit, and an analysis condition output unit for retrieving and outputting an analysis condition for analyzing the analysis target metabolite identified by the metabolite identification unit with the mass spectrometer from the analysis condition storage unit.

## Description

### CROSS-REFERENCE TO RELATED APPLICATIONS

This application claims priority under 35 U.S.C. §119 to Japanese Patent Application No. 2023-171601 filed on October 2, 2023, the entire disclosure of which is incorporated herein by reference in its entirety.

### BACKGROUND OF THE INVENTION

### Field of the Invention

The present disclosure relates to a metabolite analysis support system.

### Description of the Related Art

The following description sets forth the inventor's knowledge of the related art and problems therein and should not be construed as an admission of knowledge in the prior art.

Metabolites have conventionally been analyzed using a gas chromatograph-mass spectrometer (hereafter referred to as "GC/MS") or a liquid chromatograph-mass spectrometer (hereafter referred to as "LC/MS"). Metabolism is a set of chemical reactions in a body of an organism. Among them, the metabolism related to the synthesis or conversion of high-molecular compounds, such as DNA, RNA, proteins, carbohydrates, or lipids, and their constituent units (e.g., nucleic acids, amino acids, monosaccharides, sugar phosphates, fatty acids, and organic acids), which are essential components for the life support, growth, or reproduction of an organism, is referred to as primary metabolism. Metabolism other than primary metabolism is referred to as secondary metabolism. Primary metabolism includes, for example, major metabolic pathways, such as glycolysis, a TCA circuit (citric acid circuit), an electron transfer system, and a pentose phosphate pathway. The intermediate products and end products in such metabolic pathways are called primary metabolites.

The method of comprehensively analyzing the types and concentrations of various metabolites contained in a body of an organism is called metabolomics (metabolome analysis), and it is expected to be applied in various fields. For example, in the medical field, it can be applied to the search for biomarkers for early detection of diseases and the identification of disease-causing substances. In the food industry, it can be applied to quality evaluation and quality prediction, such as comparing products between manufacturers and comparing the origins of raw materials, or to the search for functional ingredients.

However, there are several thousand types of metabolites that are the targets of metabolomics, which is a much larger number as compared with genes which are the targets of genomics, or proteins which are the targets of proteomics. Although a large number of metabolites can be comprehensively analyzed by using GC/MS and LC/MS, it is not possible to analyze all metabolites simultaneously under the same conditions. Further, since there are numerous metabolites, it is time-consuming to analyze the analysis results of the metabolites.

### Prior Art Document

### Patent Document

Patent Document 1: Japanese Unexamined Patent Application Publication No. 2022-066655

### SUMMARY OF THE INVENTION

The preferred embodiments of the present disclosure have been developed in view of the above-mentioned and/or other problems in the related art. The preferred embodiments of the present disclosure can significantly improve upon existing methods and/or apparatuses.

The purpose of the present disclosure is to expedite the analysis of metabolites contained in a sample using a mass spectrometer.

The metabolite analysis support system according to the present disclosure, which has been made to solve the above-described problems, is a metabolite analysis support system for supporting the analysis of metabolites using a mass spectrometer.

The system comprises:
an enzyme information storage unit configured to store enzyme information for one or more enzymes involved in metabolic pathways in a body of an organism, the enzyme information representing a relation between the enzyme and metabolites, the metabolites including a substrate on which the enzyme acts and a resulting product;
an analysis condition storage unit configured to store an analysis condition for analyzing the metabolites with the mass spectrometer;
a gene locus information input unit configured to input information regarding a gene locus that affects an expression of an enzyme of interest;
a metabolite identification unit configured to identify the metabolites as analysis target metabolites, based on information regarding the gene locus input to the gene locus information input unit and information stored in the enzyme information storage unit, the metabolites including a substrate on which the enzyme acts and the resulting product, the enzyme being affected by the gene loci; and
an analysis condition output unit configured to retrieve an analysis condition for analyzing the analysis target metabolites identified by the metabolite identification unit with the mass spectrometer from the analysis condition storage unit, and to output the analysis condition.

In the metabolite analysis support system according to the present disclosure, information related to a gene locus that affects the expression of an enzyme of interest to the user is input to the gene locus input unit. Then, based on the above-described information and the enzyme information stored in the enzyme information storage unit, the metabolites, which are a substrate on which the enzyme affected by the gene locus acts and the resulting product, are identified, and the analysis conditions for analyzing the metabolites with a mass spectrometer are output.

Examples of enzymes of interest to a user include enzymes involved in the production of metabolites associated with a particular disease or a particular constitution of humans or enzymes involved in the production of metabolites associated with useful traits in plants and animals. In other words, the present disclosure makes it possible to target metabolites that are subjects of metabolome-QTL analysis and perform mass spectrometry analysis under the analysis conditions suitable for the metabolites. Therefore, it is possible to expedite the analysis of metabolites contained in the sample collected from an organism using a mass spectrometer.

The above and/or other aspects, features and/or advantages of various embodiments will be further appreciated in view of the following description in conjunction with the accompanying figures. Various embodiments can include and/or exclude different aspects, features and/or advantages where applicable. In addition, various embodiments can combine one or more aspects or features of other embodiments where applicable. The descriptions of aspects, features and/or advantages of particular embodiments should not be construed as limiting other embodiments or the claims.

### BRIEF DESCRIPTION OF THE DRAWINGS

The preferred embodiments of the present disclosure are shown by way of example, and not limitation, in the accompanying figures.
FIG. 1 is a block diagram of one example in which a metabolite analysis support system according to the present disclosure is applied to a metabolite analysis system.
FIG. 2 is a diagram showing an example of an analysis condition setting screen.

### DETAILED DESCRIPTION OF THE PREFERRED EMBODIMENTS

In the following paragraphs, some preferred embodiments of the present disclosure will be described by way of example and not limitation. It should be understood based on this disclosure that various other modifications can be made by those skilled in the art based on these illustrated embodiments.

Many traits of various organisms, including humans, are determined by a combination of multiple genes (gene loci). Such traits are collectively referred to as quantitative traits, and the gene loci that determine the quantitative traits are referred to as quantitative trait loci (QTLs). Some quantitative traits are affected by multiple QTLs. Therefore, once the QTLs that affect useful quantitative traits of an organism are known, it is possible to extract useful individuals using DNA markers that are highly relevant to the QTLs. Analyzing the QTLs that affect certain quantitative traits, the effects of the QTLs, the number of the QTLs, etc., from the nucleotide sequence of the DNA of an organism is referred to as QTL analysis.

In QTL analysis, a next-generation sequencer, which can analyze genomic information on DNA fragments at high speed, is highly effective, and QTLs that affect a variety of traits have been identified. In an analysis using a next-generation sequencer, DNA is extracted from a sample collected from a target organism, and this DNA is fragmented to achieve an appropriate narrow size distribution to prepare a library. The library is then submitted to a next-generation sequencer to perform sequencing of the original DNA nucleotide sequence.

The nucleotide sequence (the entire sequence) of the DNA determined by a next-generation sequencer can be analyzed using, for example, a homology search program called BLAST (Basic Local Alignment Search Tool). In an analysis using BLAST, the DNA nucleotide sequence of a target organism is compared with a nucleotide sequence database of the same species as the target organism to extract homologous regions of sequences, and the statistical significance (homology score) of these regions is calculated. Therefore, the user can identify regions of high homology and regions of low homology based on the magnitude of the homology score.

The regions of high homology are regions common to the species, while the regions of low homology are regions unique (specific) to the target organism. Accordingly, in cases where the target organism has characteristic traits that are not found in the species, regions of low homology can be presumed to be regions related to the above-mentioned characteristic traits. Therefore, by focusing on a nucleotide sequence in a region of low homology and performing QTL analysis using the nucleotide sequence as an index for an enzyme that is a protein, it is possible to estimate which enzyme's expression is affected by the QTL corresponding to the nucleotide sequence.

For a QTL analysis of a nucleotide sequence in a predetermined region, for example, the Reference Sequence (RefSeq) database, which is an open-source database of the U.S. National Center for Biotechnology Information (NCBI), can be used. The RefSeq database stores data on genomic DNA, gene transcripts, and the proteins that result from these transcripts for major organisms, from viruses to bacteria to eukaryotes. In other words, by using the RefSeq database to look up the identification information (RefSeqID) of the QTL corresponding to the nucleotide sequence, it is possible to acquire information on the protein associated with the QTL.

When the protein associated with the QTL is an enzyme in metabolic pathways, it can be presumed that the QTL affects the metabolites, which are the substrates on which the enzyme acts and the resulting products. For this reason, in this disclosure, the metabolites contained in the sample collected from an organism are analyzed by mass spectrometry by targeting the above-mentioned metabolites.

Specifically, the present disclosure is directed to a metabolite analysis support system for supporting analysis of metabolites using a mass spectrometer, the system comprises:
an enzyme information storage unit configured to store enzyme information for one or more enzymes involved in metabolic pathways in a body of an organism, the enzyme information representing a relation between the enzyme and metabolites, the metabolites including a substrate on which the enzyme acts and a resulting product;
an analysis condition storage unit configured to store an analysis condition for analyzing the metabolites with the mass spectrometer;
a gene locus information input unit configured to input information regarding a gene locus that affects an expression of an enzyme of interest;
a metabolite identification unit configured to identify the metabolites as analysis target metabolites, based on information regarding the gene locus input to the gene locus information input unit and information stored in the enzyme information storage unit, the metabolites including a substrate on which the enzyme acts and the resulting product, the enzyme being affected by the gene locus; and
an analysis condition output unit configured to retrieve an analysis condition for analyzing the analysis target metabolites identified by the metabolite identification unit with the mass spectrometer from the analysis condition storage unit, and to output the analysis condition

Here, the "enzyme of interest" corresponds to the enzyme that is influenced by the QTLs associated with given quantitative traits, as revealed by QTL analysis. In the metabolite analysis support system according to the present disclosure, when information on gene loci that affects the expression of an enzyme of interest to the user is input to the gene locus input unit, metabolites, which are the substrate on which the enzyme acts and resulting products, which are affected by the gene loci, are identified, based on the information and the enzyme information stored in the enzyme information storage unit, and the analysis conditions for analyzing the metabolites with a mass spectrometer are output. Examples of enzymes of interest to a user include enzymes involved in the production of metabolites associated with specific human diseases or constitutions, or enzymes involved in the production of metabolites associated with useful traits in plants and animals. In other words, in the present disclosure, it becomes possible to conduct a mass spectrometry analysis under the analysis conditions appropriate for the metabolites by targeting metabolites that are the subject of metabolome-QTL analysis.

### (Examples)

Hereinafter, a specific example in which the present disclosure is applied to a metabolite analysis system will be described with reference to the attached drawings.

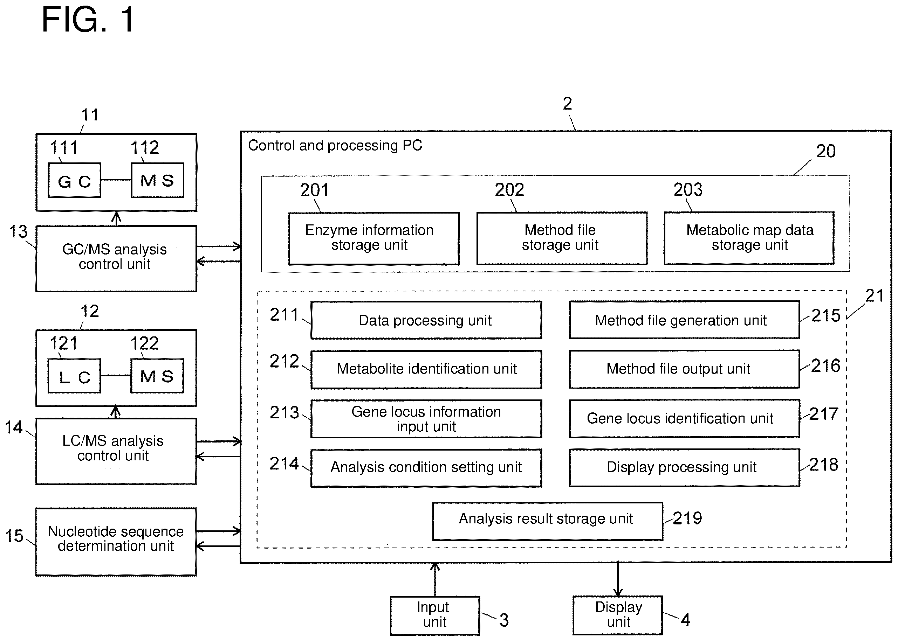

FIG. 1 is an overall schematic diagram showing the metabolite analysis system according to an embodiment of the present disclosure. This metabolite analysis system is equipped with a GC/MS 11, a GC/MS analysis control unit 13, an LC/MS analysis control unit 14, a nucleotide sequence determination unit 15, and a control and processing personal computer (PC) 2. The GC/MS 11 is composed of a GC unit 111 and an MS unit 112, as components of a mass spectrometer. The LC/MS 12 is composed of an LC unit 121 and an MS unit 122. The GC/MS analysis control unit 13 controls the operation of the GC/MS 11. The LC/MS analysis control unit 14 controls the operation of the LC/MS 12. The nucleotide sequence determination unit 15 is specifically a DNA sequencer, particularly a next-generation sequencer.

The control and processing PC 2 includes a storage unit 20 and functional blocks. The functional blocks include a data processing unit 211, a metabolite identification unit 212, a gene locus information input unit 213, an analysis condition setting unit 214, a method file generation unit 215, a method file output unit 216, a gene locus identification unit 217, a display processing unit 218, and an analysis result storage unit 219.

The entity of the control and processing PC 2 is a general personal computer, and each of the above-described functional blocks is realized by executing a pre-installed metabolite analysis support system software 21. An input unit 3 and a display unit 4 are connected to the control and processing PC 2. In this embodiment, the method file output unit 216 corresponds to the analysis condition output unit of this disclosure, and the control and processing PC 2 corresponds to the metabolite analysis support system of this disclosure.

The storage unit 20 includes an enzyme information storage unit 201, a method file storage unit 202, and a metabolic map data storage unit 203.

The enzyme information storage unit 201 stores enzyme information for one or more enzymes. The enzyme information represents relations between an enzyme involved in metabolic pathways in a body of an organism and metabolites which are a substrate on which the enzyme acts and the resulting product. Enzyme information includes information on an enzyme and metabolites that are a substrate on which the enzyme acts and the resulting products, as well as the metabolic pathway in which the enzyme is involved, and the order of metabolisms in the metabolic pathway.

The method file storage unit 202 stores the information for generating a method file for analysis conditions (analysis name, target compound, etc.) to be used when analyzing a sample containing metabolites using the GC/MS 11 or the LC/MS 12, and analysis methods of analysis results (retention index of a known standard sample, mass spectrum, characteristic ion information (mass-to-charge ratio of ions, intensity ratio of multiple ions, etc.), chromatogram peak picking method, baseline correction method, etc.). Note that the analysis condition when analyzing a certain metabolite using the GC/MS 11 or the LC/MS 12 is not limited to one, but may be more than one. The method file storage unit 202 corresponds to the analysis condition storage unit of the present disclosure.

The metabolic map data storage unit 203 stores data constituting one or more (usually several) metabolic maps. Here, a metabolic map refers to a chart that shows metabolic pathways in a body of a human and other organisms. The metabolic map lists various compounds (metabolites), which are produced in the process of metabolism, chemical reactions, enzymes involved in metabolism, etc., so that the flow of metabolism can be understood at a glance.

Next, the procedures for setting the analysis conditions when analyzing metabolites contained in a sample with the GC/MS 11 or the LC/MS 12 using the above-described metabolite analysis system and generating a method file will be described.

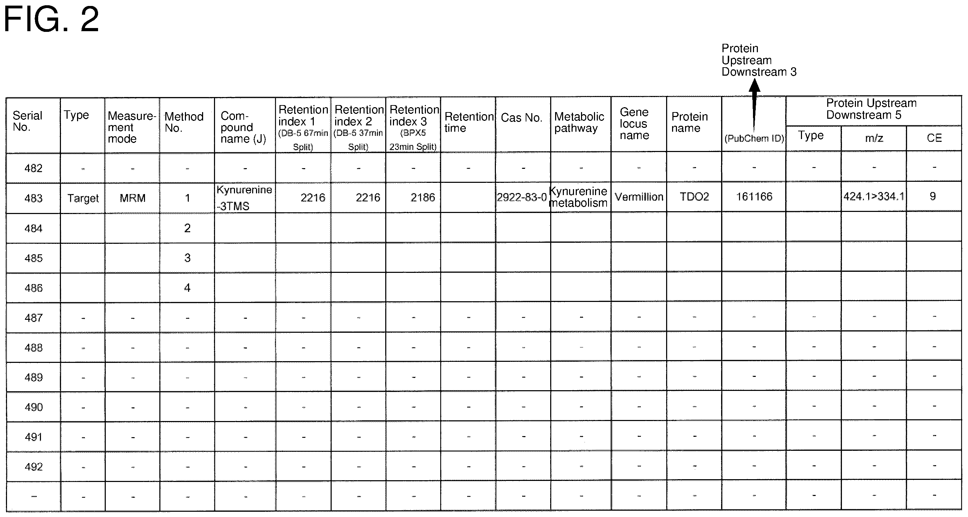

Initially, a user (worker) operates the input unit 3 to display an analysis condition setting screen. In response to this instruction, the display processing unit 218 reads out the analysis condition setting screen from the analysis condition setting unit 214 and displays the screen on the display unit 4. FIG. 2 shows an example of the analysis condition setting screen displayed on the display unit 4. On this screen, as input fields for gene locus information, displayed are an input field for each of a CAS number, a gene locus name, a protein name, and a protein identification number (PubChem ID), and a display field for displaying various conditions for mass analysis.

The user operates the input unit 3 to input predetermined gene locus information to one or more of the multiple input fields for gene locus information on the above-described analysis condition setting screen. The gene locus information input to the analysis condition setting screen is input to the gene locus information input unit 213. Note that the predetermined gene locus information refers to identification information of a gene locus (QTL) that affects the expression of an enzyme related to a metabolite that is to be checked whether or not it is contained in the sample, identification information on the enzyme, etc. The enzyme associated with the metabolites whose presence or absence in the sample needs to be confirmed corresponds to the "enzyme of interest" in the present disclosure.

The input fields for the gene locus information include a metabolic pathway selection field for selecting a metabolic pathway. When the metabolic pathway selection field is selected by operating the input unit 3, names of multiple metabolic pathways are displayed as a pull-down list. Therefore, from this pull-down list, the metabolic pathway in which the above-described "metabolites whose presence or absence in the sample needs to be confirmed" is produced can be selected. Further, when a metabolic pathway is selected in the metabolic pathway selection field, names of proteins that are multiple enzymes involved in the metabolic pathways are displayed as a pull-down list in the protein name input field. In this case, it is possible to select a certain protein name from the pull-down list to input it.

Of course, it is also possible for the user to input gene locus information to one or more of the input fields for gene locus information using letters or symbols by operating a keyboard or other means. Further, it may be configured such that when gene locus information is input to one of the input fields for gene locus information, other information (e.g., protein name, PubChem ID) associated with the information (e.g., gene locus name) is automatically displayed in the input field.

The gene locus information can also be acquired from the nucleotide sequence determined by extracting the DNA contained in the sample and analyzing the genomic information on the DNA using the nucleotide sequence determination unit 15 (next-generation sequencer). That is, when the user operates the input unit 3 to instruct an input of the information on nucleotide sequence from the nucleotide sequence determination unit 15 to the gene locus identification unit 217 and to input the enzyme of interest, the gene locus identification unit 217 acquires the gene locus information from the nucleotide sequence determination unit 15. Then, the gene locus identification unit 217 performs QTL analysis on the acquired nucleotide sequence using the enzyme as an index to identify the gene locus affecting the expression of the enzyme described above. The gene locus identified by the gene locus identification unit 217 is input into the gene locus information input unit 213.

The analysis condition setting screen shown in FIG. 2 represents the state in which "kynurenine metabolism" is input as the metabolic pathway, "Vermillion" as the gene locus name, "TD02" as the protein name, "161166" as the PubChem ID, and "2922-83-0" as the Cas number.

When gene locus information is input to the input field of the gene information, the metabolite identification unit 212 reads out the metabolites, which are the substrates on which the enzyme acts and the resulting products associated with the gene locus, from the enzyme information storage unit 201, and identifies these two metabolites as the analysis target metabolites. Further, in this example, the metabolite identification unit 212 reads out the metabolites that are substrates on which the enzyme acts and the resulting products associated with the gene locus, as well as five metabolites that are located downstream from the substrate on which the enzyme acts and five metabolites that are located upstream from the products, and identifies these metabolites as the target metabolites for analysis.

Note that in the case where the number of metabolites located on the downstream or upstream side is less than 5, the number of metabolites to be identified as the analysis target metabolites will be less than 5. In other words, in this example, when gene locus information is input, up to 12 metabolites are identified as analysis target metabolites. Further, with this, the names of up to 12 identified analysis target metabolites are displayed sequentially in the list of compound names on the analysis condition setting screen.

Note that although the number of analysis target metabolites is set to a maximum of 12, the number of analysis target metabolites may be as few as two (i.e., only metabolites that are a substrate on which the enzyme acts and the resulting products associated with the gene locus) or three or more. Further, it may be configured such that the user can operate the input unit 3 to specify the number of metabolites to be included in the analysis target metabolites, in addition to the metabolites that are the substrates on which the enzyme acts on and the resulting products associated with the gene locus. In this configuration, the input unit 3 corresponds to the metabolite number specification unit of the present disclosure.

Once the analysis target metabolites are identified by the metabolite identification unit 212, the analysis condition setting unit 214 reads out and sets the information on analysis conditions, methods of analysis results, etc., to be used when analyzing a sample containing each analysis target metabolite using the GC/MS 11 or the LC/MS 12 from the method file storage unit 202. Further, the method file generation unit 215 generates a method file that describes the procedure (schedule) for analyzing each analysis target metabolite, the analysis conditions, and the method for analyzing the analysis results. The generated method file is stored in the method file output unit 216 along with its identification information (e.g., method number, file name, etc.).

When the identification information on the method file stored in the method file output unit 216 is selected by the user, the method file output unit 216 reads out the method file selected by the selection instruction and outputs it to the GC/MS analysis control unit 13 or the LC/MS analysis control unit 14. When the GC/MS analysis control unit 13 or the LC/MS analysis control unit 14 receives an instruction to start analysis, it controls the GC/MS 11 or the LC/MS 12 according to the method file and executes the analysis of the sample.

Since the analysis conditions suitable for each analysis target metabolite are set in the above-described method file, the analysis target metabolite can be detected with high accuracy and reliability by performing the analysis of the sample under these analysis conditions. Further, the analysis is performed by targeting specific analysis target metabolites among many metabolites contained in the sample, thus shortening the analysis time and enabling rapid analysis.

The analysis data collected by the GC/MS 11 and/or the LC/MS 12 is input to the data processing unit 211. The data processing unit 211 performs data processing based on the data analysis method described in the method file. In this method file, the retention index, the mass spectrum, and the characteristic ion information are established for each analysis target metabolite. Therefore, by using this information, it is possible to reliably acquire the quantitative value of the analysis target metabolite. Once the quantitative values are obtained for all analysis target metabolites, the analysis results are stored in the analysis result storage unit 219.

Note that after the quantitative values of the analysis target metabolites are acquired, the user operates the input unit 3 to generate a metabolic map. With this, the display processing unit 218 reads out the metabolic map data from the metabolic map data storage unit 203, reads out the measurement results (quantitative values) of the metabolites listed in the metabolic map from the analysis result storage unit 219, and fills them in the corresponding locations on the metabolic map. From such a metabolic map, the user can easily recognize whether or not the analysis target metabolite is contained in the sample, and if so, in what quantity.

### [Aspects]

It is clear to those skilled in the art that the exemplary embodiments described above are specific examples of the following aspects.

### (Item 1)

A metabolite analysis support system according to one aspect of the present invention is a metabolite analysis support system for supporting an analysis of metabolites using a mass spectrometer. The system comprises:
an enzyme information storage unit configured to store enzyme information for one or more enzymes involved in metabolic pathways in a body of an organism, the enzyme information representing a relation between the enzyme and metabolites, the metabolites including a substrate on which the enzyme acts and a resulting product;
an analysis condition storage unit configured to store an analysis condition for analyzing the metabolites with the mass spectrometer;
a gene locus information input unit configured to input information regarding a gene locus that affects an expression of an enzyme of interest;
a metabolite identification unit configured to identify the metabolites as analysis target metabolites, based on information regarding the gene locus input to the gene locus information input unit and information stored in the enzyme information storage unit, the metabolites including a substrate on which the enzyme acts and the resulting product, the enzyme being affected by the gene locus; and
an analysis condition output unit configured to retrieve an analysis condition for analyzing the analysis target metabolites identified by the metabolite identification unit with the mass spectrometer from the analysis condition storage unit, and to output the analysis condition

According to the metabolite analysis support system as recited in the above-described Item 1, based on the information on the gene locus that affects the expression of the enzyme of interest to the user and the enzyme information stored in the enzyme information storage unit, it is possible to identify the metabolites, which are the substrates and products of the enzyme affected by the gene locus, and to obtain the analysis conditions for analyzing those metabolites using a mass spectrometer. Therefore, by inputting enzymes involved in the production of metabolites related to a specific human disease or a specific constitution, enzymes involved in the production of metabolites related to useful traits in plants and animals, etc., as enzymes of interest to the user, it is possible to target metabolites, which are the target of metabolome-QTL analysis, and analyze them under the analytical conditions suitable for these metabolites.

### (Item 2)

The metabolite analysis support system as recited in the above-described Item 2 may be configured such that in the metabolite analysis support system as recited in the above-described Item 1, the system further includes:
a gene locus identification unit configured to perform QTL analysis of a nucleotide sequence of DNA contained in a sample collected from the organism, using the enzyme of interest as an index, to identify the gene locus that affects the expression of the enzyme.

According to the metabolite analysis support system as recited in the above-described Item 2, it is possible to easily obtain the information on the gene locus that affects the expression of enzymes of interest. In the metabolite analysis support system according to the present disclosure, information related to loci that affect the expression of enzymes of interest to the user is input to the locus information input unit.

### (Item 3)

The metabolite analysis support system as recited in the above-described Item 3 may be configured such that in the metabolite analysis support system as recited in the above-described Item 2, the system may be configured to further include:
an input instruction unit configured to input information on the gene locus identified by the gene locus identification unit to the gene locus information input unit.

According to the metabolite analysis support system as recited in the above-described Item 3, it is possible to simplify the process of inputting the information on the gene locus that affects the expression of the enzyme of interest, as identified by the gene locus identification unit, to the gene locus information input unit.

### (Item 4)

The metabolite analysis support system as recited in the above-described Item 4 may be configured such that in the metabolite analysis support system as recited in the above-described Item 2 or 3, the system further includes:
a nucleotide sequence determination unit configured to determine the nucleotide sequence of DNA contained in the sample collected from the organism.

According to the metabolite analysis support system as recited in the above-described Item 4, it is possible to easily perform a series of tasks from the determination of the nucleotide sequence of the DNA contained in the sample to the identification of the gene locus that affects the expression of the enzyme of interest.

### (Item 5)

The metabolite analysis support system as recited in the above-described Item 5 may be configured such that metabolite analysis support system as recited in the above-described Item 4, the nucleotide sequence determination unit is a DNA sequencer.

In this case, the use of a DNA sequencer, referred to as a next-generation sequencer, is particularly desirable because it can analyze the genomic information of DNA at high speed and determine its nucleotide sequence.

### (Item 6)

The metabolite analysis support system as recited in the above-described Item 6 may be configured such that, in the metabolite analysis support system as recited in any one of the above-described Items 1 to 5,
the enzyme information storage unit stores the enzyme information in an order of the metabolic pathways, and
the metabolite identification unit identifies a predetermined number of metabolites on a downstream or upstream side in the metabolic pathways relative to a reaction of the enzyme affected by the gene locus, as the analysis target metabolites.

### (Item 7)

The metabolite analysis support system as recited in the above-described Item 7 may be configured such that in the metabolite analysis support system as recited in the above-described Item 6, the system further includes:
a metabolite number specification unit configured to specify the number of metabolites on the downstream or upstream side, wherein the number of metabolites is to be included in the analysis target metabolites.

According to the metabolite analysis support system as recited in the above-described Item 6 or 7, it is possible to easily perform mass analysis by expanding the analysis target to the vicinity of the metabolic pathway in which the enzyme of interest is involved.

### Description of Reference Symbols

- 11:: GC/MS
- 111:: GC unit
- 112:: MS unit
- 12:: LC/MS
- 121:: LC unit
- 122:: MS unit
- 13:: GC/MS analysis control unit
- 14:: LC/MS analysis control unit
- 15:: Nucleotide sequence determination unit
- 2:: Control and processing PC
- 20:: Storage unit
- 201:: Enzyme information storage unit
- 202:: Method file storage unit
- 203:: Metabolic map data storage unit
- 21:: Metabolite analysis support system software
- 211:: Data processing unit
- 212:: Metabolite identification unit
- 213:: Gene locus information input unit
- 214:: Analysis condition setting unit
- 215:: Method file generation unit
- 216:: Method file output unit
- 217:: Gene locus identification unit
- 218:: Display processing unit
- 219:: Analysis result storage unit
- 3:: Input unit
- 4:: Display unit

## Claims

1. A metabolite analysis support system for supporting analysis of metabolites using a mass spectrometer, comprising:
an enzyme information storage unit configured to store enzyme information for one or more enzymes involved in metabolic pathways in a body of an organism, the enzyme information representing a relation between the enzyme and metabolites, the metabolites including a substrate on which the enzyme acts and a resulting product;
an analysis condition storage unit configured to store an analysis condition for analyzing the metabolites with the mass spectrometer;
a gene locus information input unit configured to input information regarding a gene locus that affects an expression of an enzyme of interest;
a metabolite identification unit configured to identify the metabolites as analysis target metabolites, based on information regarding the gene locus input to the gene locus information input unit and information stored in the enzyme information storage unit, the metabolites including a substrate on which the enzyme acts and the resulting product, the enzyme being affected by the gene locus; and
an analysis condition output unit configured to retrieve an analysis condition for analyzing the analysis target metabolites identified by the metabolite identification unit with the mass spectrometer from the analysis condition storage unit, and to output the analysis condition.

2. The metabolite analysis support system as recited in claim 1, further comprising:
a gene locus identification unit configured to perform QTL analysis of a nucleotide sequence of DNA contained in a sample collected from the organism, using the enzyme of interest as an index, to identify the gene locus that affects the expression of the enzyme.

3. The metabolite analysis support system as recited in claim 2, further comprising:
an input instruction unit configured to input information on the gene locus identified by the gene locus identification unit to the gene locus information input unit.

4. The metabolite analysis support system as recited in claim 2, further comprising:
a nucleotide sequence determination unit configured to determine the nucleotide sequence of DNA contained in the sample collected from the organism.

5. The metabolite analysis support system as recited in claim 4,
wherein the nucleotide sequence determination unit is a DNA sequencer.

6. The metabolite analysis support system as recited in claim 1,
wherein the enzyme information storage unit stores the enzyme information in an order of the metabolic pathways, and
wherein the metabolite identification unit identifies a predetermined number of metabolites on a downstream or upstream side in the metabolic pathways relative to a reaction of the enzyme affected by the gene locus, as the analysis target metabolites.

7. The metabolite analysis support system as recited in claim 6, further comprising:
a metabolite number specification unit configured to specify the number of metabolites on the downstream or upstream side, wherein the number of metabolites is to be included in the analysis target metabolites.
